**Europäisches Patentamt**

**European Patent Office**

**Office européen des brevets**

(19)

(11) Veröffentlichungsnummer : **0 240 484 B1**

(12) **EUROPÄISCHE PATENTSCHRIFT**

(45) Veröffentlichungstag der Patentschrift :
**15.01.92 Patentblatt 92/03**

(21) Anmeldenummer : **87890046.3**

(22) Anmeldetag : **10.03.87**

(51) Int. Cl.⁵ : **A61K 9/12, A61K 31/44**

(54) **Pharmazeutische Zubereitung sowie Verfahren zur Herstellung derselben.**

(30) Priorität : **19.11.86 AT 3092/86
10.03.86 AT 620/86**

(43) Veröffentlichungstag der Anmeldung :
**07.10.87 Patentblatt 87/41**

(45) Bekanntmachung des Hinweises auf die
Patenterteilung :
**15.01.92 Patentblatt 92/03**

(84) Benannte Vertragsstaaten :
**AT BE CH DE ES FR GB GR IT LI LU NL SE**

(56) Entgegenhaltungen :
**EP-A- 0 117 888
EP-A- 0 175 671
GB-A- 2 162 745
US-A- 4 442 112**

(73) Patentinhaber : **Burghart, Kurt, Dr.
Sägeberg 8
W-2217 Rosdorf (DE)**
Patentinhaber : **Burghart, Walter
Salmgasse 4
A-1030 Wien (AT)**

(72) Erfinder : **Burghart, Kurt, Dr.
Sägeberg 8
W-2217 Rosdorf (DE)**
Erfinder : **Burghart, Walter
Salmgasse 4
A-1030 Wien (AT)**

(74) Vertreter : **Haffner, Thomas M., Dr. et al
Patentanwaltskanzlei Dipl.-Ing. Adolf
Kretschmer Dr. Thomas M. Haffner
Schottengasse 3a
A-1014 Wien (AT)**

## Beschreibung

Die Erfindung bezieht sich auf eine pharmazeutische Zubereitung mit Nifedipin als Wirkstoff sowie auf ein Verfahren zur Herstellung pharmazeutischen Zubereitung mit Nifedipin als Wirkstoff.

Nifedipin ist als Koronartherapeutikum bekannt und Nifedipin enthaltende feste Präparatzusammensetzungen sowie Verfahren zu ihrer Herstellung sind beispielsweise aus der DE-OS 28 22 882 bekannt geworden. Bei Nifedipin handelt es sich um 4-(2-nitrophenyl)-2,6-dimethyl-3,5-dicarbomethoxy-1,4-dihydropyridin und es ist weiters bereits bekannt, daß diese Verbindung in hohem Maße lichtempfindlich ist. Es sind daher bereits eine Reihe von Vorschlägen gemacht worden, wie die Verabreichung dieser Verbindung erfolgen soll, um die mit der Zersetzung unter Lichteinfluß verbundene Abnahme der Wirksamkeit bzw. den Verlust an Wirksamkeit zu beseitigen. Im besonderen ist aus der DE-OS 28 22 882 eine Präparatzusammensetzung zu entnehmen, bei welcher Nifedipin als feste Präparatzusammensetzung mit Polyvinylpyrrolidon Methylzellulose, Hydroxypropylzellulose und Hydroxypropylmethylzellulose vermengt wird, wobei zusätzlich verschieden oberflächenaktive Mittel, insbesondere öle, angewandt wurden. Diese Verabreichungsform wurde in erster Linie im Hinblick auf die Verbesserung der Verfügbarkeit des Arzneistoffes zur Resorption gewählt und ist weiterhin mit erheblichen Nachteilen bezüglich der Stabilität unter Lichteinfluß verbunden. Zur oralen Verabreichung sind neben Tabletten und Pillen auch schon Kapseln vorgeschlagen worden, wobei bei diesen Kapseln Farbstoffe in die Kapselhülle einzuarbeiten sind, um die Lichtzersetzung hintanzuhalten. Eine derartige Zusammensetzung ist beispielsweise der DE-PS 22 09 526 zu entnehmen, wobei als Farbstoff Gelb-Orange S 15985 neben einem Opakisierungsmittel in der Kapselhülle gewählt wurde. Derartige Kapselhüllen sind in der Regel temperatur- und feuchtigkeitsempfindlich, und es ist auch in diesem Fall eine vollständige Sicherheit gegen Zersetzung der wirksamen Substanz nicht gegeben.

Die EP-A-0 175 671 beschreibt lagerstabile pharmazeutische Zubereitungen mit Nifedipin als Wirkstoff, in welchem neben dem Wirkstoff ein Lösungsmittel bzw. Lösungsmittelgemische sowie gegebenenfalls ein Treibmittel enthalten ist.

Flüssigzubereitungen mit Dihydropyridinen, wie beispielsweise Tropflösungen, sind beispielsweise der DE-OS 33 07 422 sowie der korrespondierenden EP-A zu entnehmen. Diese flüssigen pharmazeutischen Zubereitungen mit Nifedipin zeigen bei sublingualer Anwendung durchwegs zwar einen relativ raschen Wirkungseintritt, aber nur eine relativ geringe Plasmakonzentration in der ersten Phase nach der Verabreichung. Die Verabreichung derartiger Flüssigzubereitungen erfolgt gemäß der DE-OS 33 07 422 und der EP-A-0 117 888 ohne Anwendung von Treibgas.

Aus dem International Journal of Clinical Pharmacology, Therapy and Toxicology, Vol. 24, Nr. 6, 1986, Seiten 283 bis 286, mit dem Titel "Nifedipine serum concentrations following sublingual and oral doses" von G.R.Brown's, D.G.Fraser, J.A.Castile, P.Gaudreault, D.R.Platt und P.A.Friedman ist eine Gegenüberstellung der Wirkungsweise von Nifedipin bei oraler und sublingualer Applikation zu entnehmen. Gemäß dieser Arbeit wurde beobachtet, daß die orale Verabreichung zu höheren Plasmamaxima und damit zu einer sehr guten Wirkung führt, wohingegen die sublinguale Verabreichung sich durch einen raschen Wirkungseintritt bei verringerten Plasmamaxima auszeichnet. Diese Wirkungsunterschiede wurden darauf zurückgeführt, daß bei sublingualer Verabreichung zunächst eine Ausfällung von Nifedipinkristallen mit dem Speichel auftritt und somit nur ein Teil des verabreichten Nifedipins resorbiert wird, wohingegen der weitere Anteil des Nifedipins, welcher in Form von Kristallen ausgefällt wurde, erst in der Folge aufgelöst wird und in die Blutbahn gelangt.

Die Erfindung zielt nun darauf ab, eine pharmazeutische Zubereitung der eingangs genannten Art zu schaffen, welche sich gegenüber den bekannten Rezepturen durch eine raschere Resorption und eine hohe Plasmakonzentration innerhalb kurzer Zeit nach sublingualer Applikation auszeichnet. Gleichzeitig soll die erfindungsgemäße Zubereitung ein hohes Maß an Stabilität aufweisen und uneingeschränkt und ohne Zuhilfenahme ärztlicher Hilfe rasch verabreicht werden können. Zur Lösung dieser Aufgabe besteht die erfindungsgemäße pharmazeutische Zubereitung im wesentlichen darin, daß der Wirkstoff zusammen mit 1) Polyalkoholen, wie Polyäthylen-und/oder Polyalkylenglykolen und/oder Alkylphenyläthern des Polyäthylenglykols und-/oder Polyglycerinfettsäureestern und/oder Glycerin-Polyäthylenglykoloxyfettsäureestern wie Glycerin-Polyäthylenglykoloxyoleat und/oder Glycerin-Polyäthylenglykoloxystearat und/oder partiellen Fettsäureestern des Sorbitans bzw. des Polyhydroxyäthylensorbitans und/oder Polyvinylalkohols und/oder Polyhydroxyäthylenfettalkoholäthern bzw. Polyhydroxyäthylenfettsäureestern und/oder Polyhydroxyäthylenpolyhydroxypropylenkondensaten und/oder mit 2) Copolymeren aus Acrylsäure und Methacrylsäure, und/oder Copolymeren aus Methacrylsäureestern und Acrylsäureestern, und/oder Copolymeren aus Methacrylsäure und Methacrylsäuremethylester und 3), Äthanol gelöst vorliegt, wobei das Gewichtsverhältnis von Nifedipin und den Verbindungen 1) und 2) 1:2 bis 1:25 beträgt, wobei 4) als Fällungshemmer bei Einbringung in den Speichel Copolyvidon oder Polyvinylpyrrolidon oder Copolymere aus Methacrylsäure und Acrylsäureäthylester in Mengen von 25 bis 300 Gew.-%, insbesondere 50 bis 200 Gew.-%, bezogen auf die Menge an Nifedipin oder Propylencarbonat ver-

2

EP 0 240 484 B1

wendet wird, die Zusammensetzung zusätzlich Äthanol, gegebenenfalls wenigstens teilweise ersetzt durch mittelkettige Fettsäuredi- und/oder -triglyceride im Verhältnis von 1:25 bis 1:4 Gewichtsteilen Nifedipin:Äthanol und ein Treibgas enthält und wobei weiters in der Zusammensetzung maximal 7,5 Gew.-% Wasser in bezug auf das Gesamtgewicht der Lösung enthalten sein können.

Der Term "Polyalkohole" bezeichnet Substanzen mit wenigstens zwei freien Hydroxylgruppen, insbesondere polymere Substanzen, in deren Monomereinheit wenigstens eine Hydroxylgruppe, die an der Polymerisationsreaktion nicht teilnimmt, enthalten ist.

Der Term "mittelkettige Fettsäuretriglyceride" bezeichnet Triglyceride gesättigter Fettsäuren mit Kettenlängen von 8 bis 12 Kohlenstoffatomen, insbesondere gesättigte Fettsäuren mit Kettenlängen von 8 und 10 Kohlenstoffatomen.

Neben dem Wirkstoff, dem Fällungshemmer bei Einbringung in den Speichel bzw. Lösungsmittel, Äthanol und dem Treibgas können naturgemäß weitere pharmazeutische Hilfsstoffe, insbesondere Aromastoffe, Weichmacher sowie gegebenenfalls inerte Füllstoffe, im üblichen Ausmaß eingesetzt werden. Durch die Versprühbarkeit mit einem Treibgas ergibt sich die Möglichkeit, die pharmazeutische Zubereitung vor der Applikation in einem lichtgeschützten Behältnis, beispielsweise einer Spraydose, zu lagern, so daß ein hohes Maß an Stabilität sichergestellt ist. Bei Einhalten der Gewichtsverhältnisse von Nifedipin:Lösungsmittel im angegebenen Bereich 1:2 bis 1:25 unter gleichzeitiger Einhaltung der Werte für Äthanol hat sich überraschenderweise gezeigt, daß eine stabile Lösung vorliegt, welche vor allen Dingen mit den Lösungsmitteln, nämlich den Polyalkoholen, wie Polyäthylen-und Polyalkylenglykol sowie mit den besonders bevorzugten Substanzen: Glycerin-Polyäthylenglykoloxystearat, Polyvinyl-pyrrolidon, Copolyvidon, Propylencarbonat und Copolymere aus Acrylsäureäthylester und Methacrylsäure eine überaus rasche und im Vergleich mit anderen Formulierungen wesentlich raschere Resorption ergibt. Die Wahl dieser Lösungsmittel bzw. Fällungshemmer bei Einbringung in den Speichel im angegebenen Gewichtsverhältnis relativ zu Nifedipin ermöglicht es, stabile Lösungen selbst bei hohen Mengen an Treibgas aufrechtzuerhalten, wobei sich überraschenderweise gezeigt hat, daß im Falle des Glycerin-Polyäthylenglykoloxystearats, in welchem Nifedipin vergleichsweise besser löslich ist, höhere Anteile des Glycerin-Polyäthylenglykoloxystearats bevorzugt sind, da durch diese höheren Anteile im Rahmen des angegebenen Bereiches wegen der besseren Löslichkeit eine raschere Resorption beobachtet werden konnte und dies vor allem in Kombination mit Copolyvidon. Mit Rücksicht auf die Tatsache, daß die pharmazeutische Zubereitung für das Versprühen als Aerosol formuliert ist, ist Äthanol in dem genannten Ausmaß dahingehend vorteilhaft, daß die Mischbarkeit des Lösungsmittels mit dem Treibgas ermöglicht wird. Es hat sich gezeigt, daß bei Einhalten eines Mengenverhältnisses von 1:25 bis 1:4 Nifedipin:Äthanol in Anwesenheit von Treibgas in Mengen von bis zu 40 % eine klare Lösung des Nifedipins in den genannten Lösungsmitteln, vor allen Dingen den drei erstgenannten Lösungsmitteln, festgestellt werden kann, wobei der Anteil des Alkohols bei steigenden Mengen an Lösungsmittel zurückgenommen werden kann. Ein Ersatz des Alkohols durch mittelkettige Fettsäuretriglyceride ist bei Verwendung von Propylencarbonatzusätzen als Fällungshemmer bei Einbringung in den Speichel besonders vorteilhaft. In der Zusammensetzung sind maximal 7,5 Gew.-% Wasser in bezug auf das Gesamtgewicht der Lösung enthalten, um einer Entmischung der erfindungsgemäßen Zubereitung vorzubeugen.

Flüssige pharmazeutische Zubereitungen für die Verabreichung als Aerosol enthalten Nifedipin als Wirkstoff, ein geeignetes Lösungsmittel und ein in einem Spraybehälter enthaltenes Treibmittel, wobei das Lösungsmittel Äthanol und einen Polyalkohol enthält, wobei gegebenenfalls zumindest ein Teil des Äthanols durch mittelkettige Fettsäuretriglyceride ersetzt ist, und ein oder mehrere als Fällungshemmer bei Einbringung in den Speichel wirkende Substanzen enthalten sind, die aus der Gruppe der Polyalkohole, Polyvinylpyrrolidon, Alkylencarbonate, Copolyvidon und Copolymere aus Acrylsäureäthylester und Methacrylsäure gewählt sind, wobei das Mengenverhältnis Nifedipin:Äthanol bzw. mittelkettigen Fettsäuretriglyceriden im Bereich von 1:25 bis 1:4 liegt und das Mengenverhältnis Nifedipin:Polyalkohol im Bereich von 1:2 bis 1:25 liegt, mit dem Proviso, daß

a) wenn der Polyalkohol Polyäthylenglykol ist, zusätzlich ein oder mehrere andere Polyalkohole enthalten sind und/oder ein wie oben definierter Fällungshemmer bei Einbringung in den Speichel und/oder das Äthanol zumindest teilweise durch mittelkettige Fettsäuretriglyceride ersetzt ist und

b) wenn der Polyalkohol Glycerin-Polyäthylenglykoloxystearat ist, das Gewichtsverhältnis Treibmittel:Nifedipin unter 220 liegt und/oder das Äthanol zumindest teilweise durch mittelkettige Fettsäuretriglyceride ersetzt ist und wobei weiters

c) in der Zusammensetzung maximal 7,5 Gew.-% Wasser in bezug auf das Gesamtgewicht der Lösung enthalten sein können.

Langzeitstabile Lösungen von Nifedipin nach Applikation (in der Mischung mit Wasser) werden dadurch erreicht, daß die Lösung Polyvinylpyrrolidon (PVP) und/oder Copolyvidon und/oder Copolymere aus Acrylsäureäthylester und Methacrylsäure als Fällungshemmer bei Einbringung in den Speichel enthält. Es hat sich gezeigt, daß Zusätze von Polyvinylpyrrolidon und/oder Copolyvidon, d.i. ein Copolymerisat aus Polyvinylpyr-

3

rolidon und Polyvinylacetat, und/oder Copolymere aus Acrylsäureäthylester und Methacrylsäure in Mengen von 25 bis 300 Gew.-%, insbesondere 50 bis 200 Gew,-%, bezogen auf die Menge an Nifedipin das Löslichkeitsverhalten von Nifedipin in Wasser beeinflussen, so daß in der flüssigen Zubereitung ein für sublinguale Verabreichung charakteristischer rascher Wirkungseintritt und gleichzeitig eine hohe Plasmakonzentration erzielt werden kann. Die Menge an PVP und/oder Copolyvidon und/oder Copolymere aus Acrylsäureäthylester und Methacrylsäure soll hiebei etwa das Ein- bis Zweifache der Menge des Wirkstoffes betragen. Andere Zusätze, wie beispielsweise Harnstoff, Hydroxypropylmethylcellulose und einige andere Polymere, welche als pharmakologisch derzeit unbedenklich gelten, haben diesen überraschenden Effekt nicht zutage gebracht.

Bei der Verabreichung von erfindungsgemäßen Flüssigzubereitungen, in welchen sowohl Polyvinylpyrrolidon als auch Äthanol enthalten ist, konnte ein besonders rascher Wirkungseintritt und hohe Plasmakonzentrationen im Vergleich zu herkömmlichen in Form von Kapseln zu verabreichenden Zusammensetzungen mit Polyvinylpyrrolidon beobachtet werden.

Ebenfalls hohe Plasmakonzentration und ein rascher Wirkungseintritt wird bei Verwendung von Propylencarbonat als Fällungshemmer bei Einbringung in den Speichel erreicht. Derartige Zusammensetzungen sind vorzugsweise dadurch gekennzeichnet, daß bei Verwendung von Propylencarbonat als Fällungshemmer bei Einbringung in den Speichel, zur Herstellung einer stabilen, sprühfähigen Zusammensetzung, 25 bis 40 %, insbesondere 30 bis 35 Gew.-%, Treibgas enthalten sind. Bei diesen Zusammensetzungen wurde überraschenderweise festgestellt, daß bei zu hohen Treibgasmengen aus der Zusammensetzung fast augenblicklich Nifedipin ausfällt, bei zu niedrigen Treibgasmengen ist die Lösung nicht sprühfähig. Wenn allerdings Treibgasmengen von 25 bis 40 Gew.-%, insbesondere 30 bis 35 % des Gesamtgewichtes, in der Zusammensetzung enthalten sind und ein Verhältnis Nifedipin zu Propylencarbonat von 1:6 bis 1:10 sowie Nifedipin zu Fettsäuretriglyceriden von 1:6 bis 1:10 eingehalten wird, können ölige, in welchen Nifedipin im Öl gelöst bleibt und über Zeiträume von 20 min nach dem Versprühen in Wasser stabile Zusammensetzungen hergestellt werden.

Wenn neben dem Vorhandensein von Propylencarbonat als Fällungshemmer bei Einbringung in den Speichel, eine weitere als Fällungshemmer bei Einbringung in den Speichel wirkende Substanz wie Copolyvidon in der Mischung vorhanden ist, tritt eine Entmischung der öligen Zusammensetzung in eine wässerige und eine ölige Phase auf, wobei das Nifedipin in der wässerigen Phase auskristallisiert. Um derartige unerwünschte Nachteile zu vermeiden, ist bei Verwendung von Propylencarbonat zusätzlich kein Copolyvidon in der Zusammensetzung enthalten, da bei Zusammensetzungen die zusätzlich zu Propylencarbonat kein Copolyvidon enthalten, das Nifedipin in der ölphase verbleibt und nach sublingualer Applikation aus dieser resorbiert wird.

Eine wesentliche Verbesserung der oralen Resorption nach sublingualer Applikation wird daher den genannten Substanzen PVP, Copolyvidon, Copolymere aus Acrylsäureäthylester und Methacrylsäure oder Propylencarbonat, insbesondere in Kombination mit Glycerin-Polyäthylenglykoloxystearat als Lösungsmittel zugeschrieben, wobei insbesondere bei einer Kombination von Copolyvidon und Glycerin-Polyäthylenglykoloxystearat eine Nifedipin Fällung nach der Applikation bzw. Mischung mit Wasser über einen längeren Zeitraum, typisch 15 bis 20 Minuten vermieden werden kann.

Die bei üblichen Zubereitungen in Kapselform, die aus 1 Gew.-Teil Nifedipin, 15 bis 35 Gew.-Teilen Polyäthylenglykol (Durchschnittsmolekulargewicht 300 bis 600), 1 bis 10 Gew.-Teilen Glycerin bestehen, beobachtete Ausfällung von Kristallen bei sublingualer Anwendung führt zu einem gewissen Retardeffekt, welcher ein langsameres Ansteigen der Plasmawerte mit niedrigen maximalen Plasmakonzentrationen zur Folge hat, wobei allerdings bei oraler Verabreichung konventioneller Zubereitungen nach einer bestimmten Zeit ein hoher und zumeist doppelter Maximalwert der Plasmakonzentration erzielt wird. Der Zeitraum bis zum Erreichen des Maximalwertes der Plasmakonzentration bei sublingualer Anwendung konventioneller Zubereitungen beträgt typischerweise das Doppelte des Zeitraumes bis zum Erreichen eines Maximalwertes der Plasmakonzentration nach oraler Verabreichung.

Im Rahmen der vorliegenden Erfindung hat sich nun überraschenderweise gezeigt, daß bei sublingualer Applikation die Zeiträume bis zum Erreichen der Plasmamaxima umgekehrt proportional dem Zusatz an PVP und/oder Copolyvidon gesteuert werden kann, wobei erfindungsgemäß die Zubereitung so eingestellt wird, daß die Menge an PVP und/oder Copolyvidon für raschere Resorption und höhere Plasmamaxima an Nifedipin höher dosiert ist.

Als im Rahmen der vorliegenden Erfindung verwendbares Copolyvidon kann mit Vorteil ein Copolymer aus 60% Polyvinylpyrrolidon und 40% Polyvinylacetat (PVA) eingesetzt werden.

Besonders rasch wirkende, eine hohe Plasmakonzentration aufweisende und gleichzeitig langzeitstabile Zubereitungen weisen vorzugsweise eine Zusammensetzung auf, in welcher in 150 mg, 5 mg Nifedipin, 40 bis 55 mg Glycerin-Polyäthylenglykoloxystearat, 20 bis 35 mg Äthanol, 5 bis 15 mg Copolyvidon oder Polyvinylpyrrolidon oder Copolymere aus Acrylsäureäthylester und Methacrylsäure oder Gemische davon und 55 bis 70 mg Treibgas enthalten sind.

Das erfindungsgemäße Verfahren zur Herstellung einer sublingual dosierbar verabreichbaren pharmazeutischen Zubereitung mit Nifedipin als Wirkstoff besteht im wesentlichen darin, Nifedipin zusammen mit 1) Polyalkoholen, wie Polyäthylen- und/oder Polyalkylenglykolen und/oder Alkylphenyläthern des Polyäthylenglykols und/oder Polyglycerinfettsäureestern und/oder Glycerin-Polyäthylenglykoloxyfettsäureestern wie Glycerin-Polyäthylenglykoloxyoleat und/oder Glycerin-Polyäthylenglykoloxystearat und/oder partiellen Fettsäureestern des Sorbitans bzw. des Polyhydroxyäthylensorbitans und/oder Polyvinylalkohols und/oder Polyhydroxyäthylenfettalkoholäthern bzw. Polyhydroxyäthylenfettsäureestern und/oder Polyhydroxyäthylenpolyhydroxypropylenkondensaten und/oder mit 2) Copolymeren aus Acrylsäure und Methacrylsäure, und/oder Copolymeren aus Methacrylsäureestern und Acrylsäureestern, und/oder Copolymeren aus Methacrylsäure und Methacrylsäuremethylester und 3), Äthanol gelöst wird, wobei das Gewichtsverhältnis von Nifedipin und den Verbindungen 1) und 2) 1:2 bis 1:25 beträgt, wobei 4) als Fällungshemmer bei Einbringung in den Speichel Copolyvidon oder Polyvinylpyrrolidon oder Copolymere aus Methacrylsäure und Acrylsäureäthylester in Mengen von 25 bis 300 Gew.-%, insbesondere 50 bis 200 Gew.-%, bezogen auf die Menge an Nifedipin oder Propylencarbonat verwendet wird, und zusätzlich Äthanol, gegebenenfalls wenigstens teilweise ersetzt durch mittelkettige Fettsäuredi-und/oder -triglyceride im Verhältnis von 1:25 bis 1:4 Gewichtsteilen Nifedipin:Äthanol und ein Treibgas zugesetzt wird und wobei weiters in der Zusammensetzung maximal 7,5 Gew.-% Wasser in bezug auf das Gesamtgewicht der Lösung zugesetzt werden und gegebenenfalls mit Hilfsstoffen in lichtgeschützte Spraydosen gefüllt wird. Mit Polyalkoholen und dem Glycerin-Polyäthylenglykoloxystearat und den anderen genannten Lösungsmitteln lassen sich Tröpfchengrößen mit Durchmessern größer 10 μm erzielen, welche gleichfalls von Einfluß auf die rasche Resorbierbarkeit sein dürften. Im Rahmen der Erfindung können neben den pharmazeutischen Hilfsstoffen auch Geschmacksstoffe, insbesondere Pfefferminzöl od. dgl., mitverwendet werden.

Die erfindungsgemäße Untergrenze von 1:2, vorzugsweise 1:5, für das Gewichtsverhältnis Nifedipin:Lösungsmittel stellt im Rahmen der Erfindung sicher, daß eine stabile nicht zum Ausfallen neigende Lösung erzielt wird. Die erfindungsgemäß aufgefundene Obergrenze von 1:25 für das genannte Verhältnis Nifedipin:Lösungsmittel wurde im Hinblick auf die rasche Resorption und die schnellere Ansprechzeit bei sublingualer Applikation gewählt, so daß in Verbindung mit Äthanol und Treibgas keine Entmischungen auftreten.

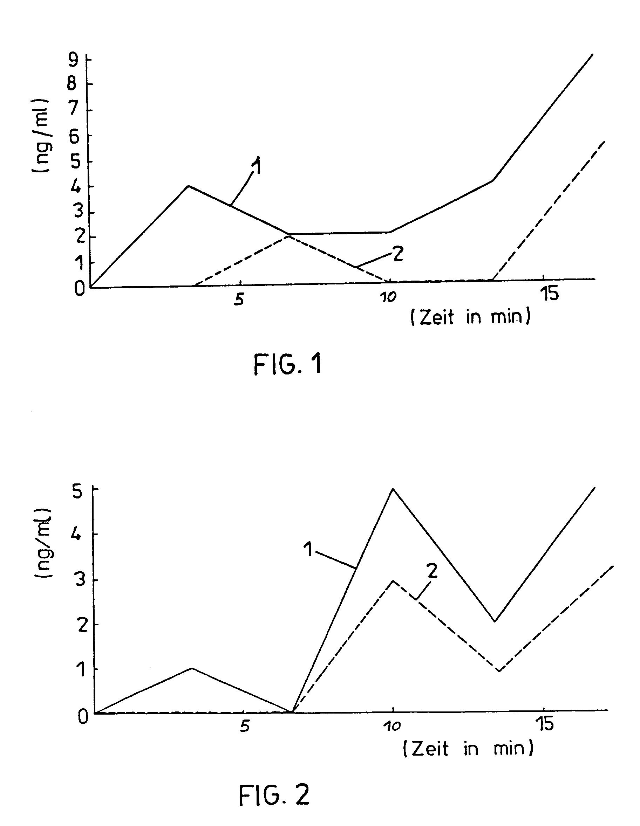

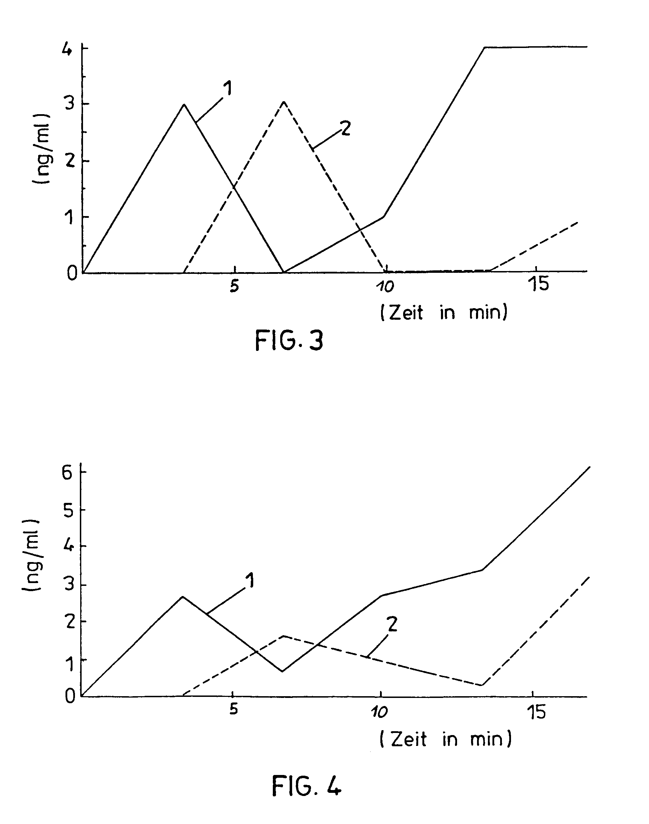

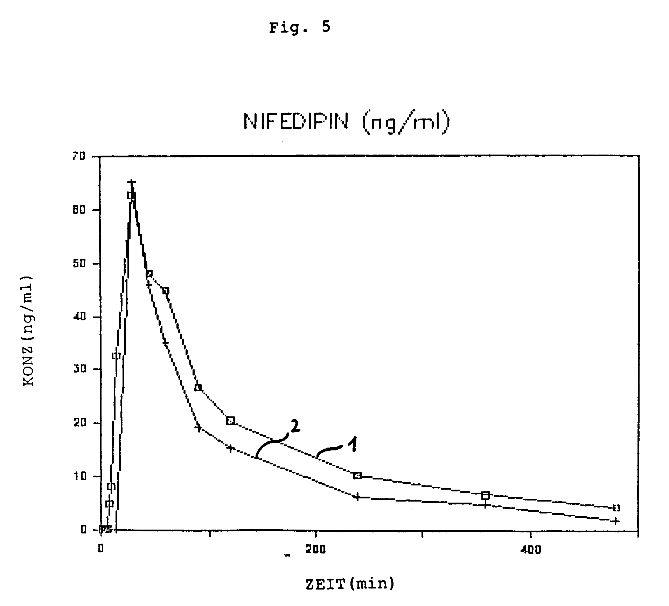

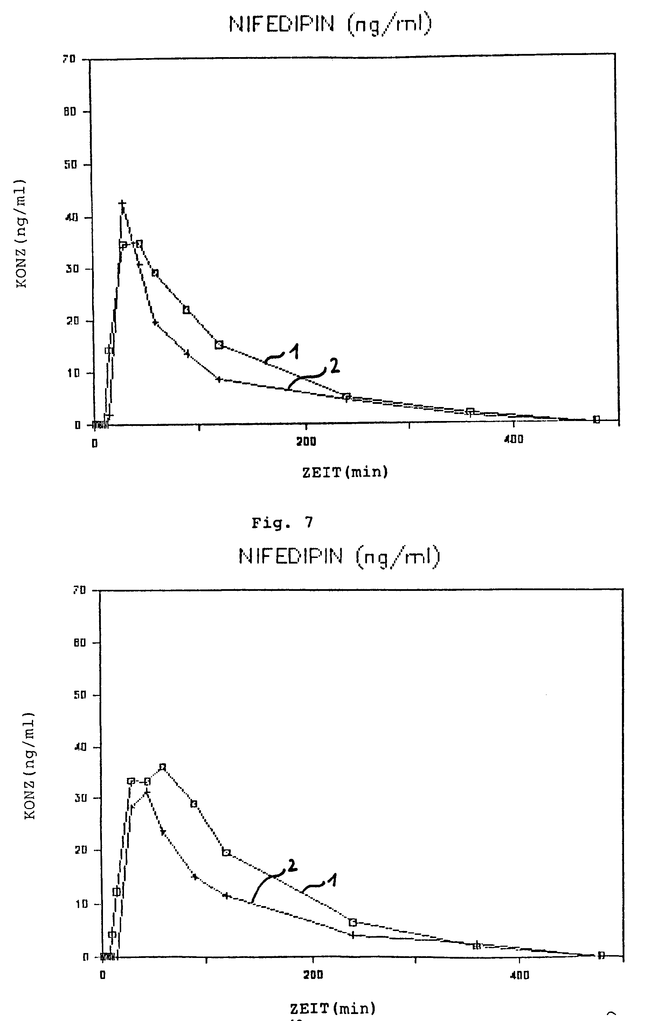

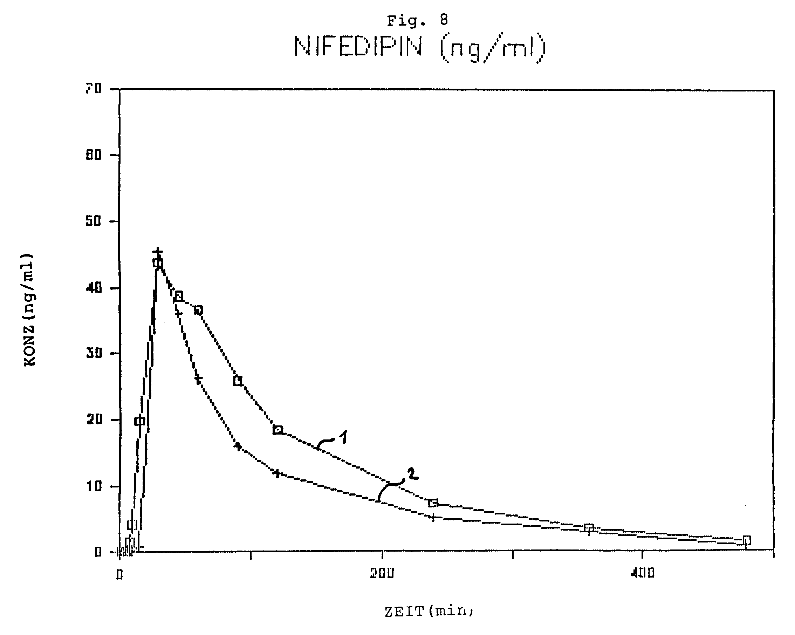

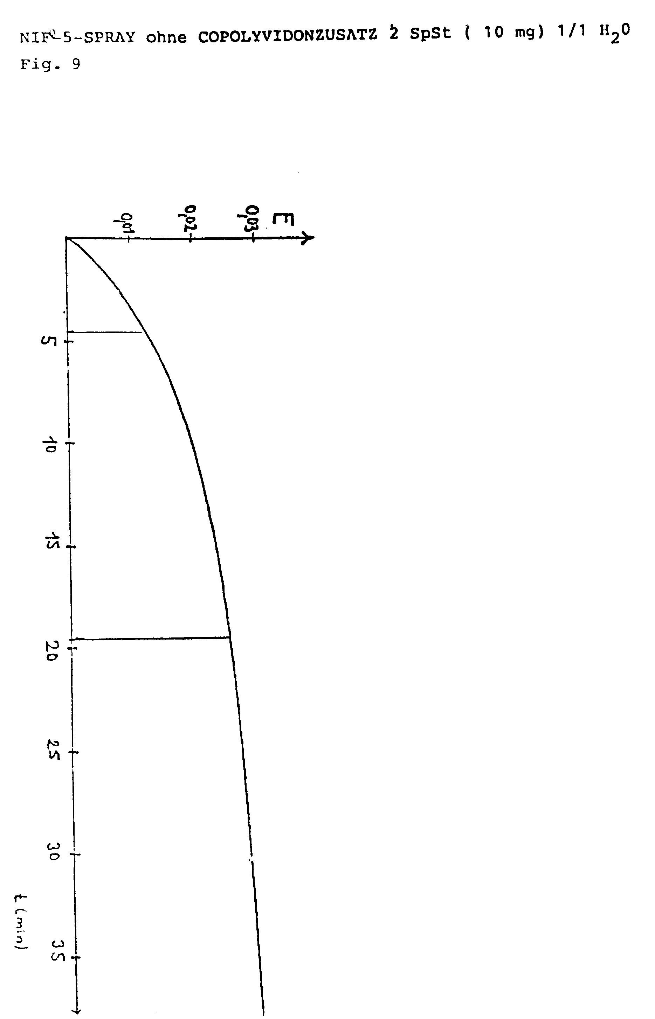

Die wesentlichen Vorteile der erfindungsgemäßen Zubereitungen gegenüber bekannten Zubereitungen ergeben sich aus den in den Zeichnungen dargestellten Vergleichsversuchen. In der Zeichnung zeigen Fig. 1 bis 3 eine vergleichende Anflutungskinetik für eine Nifedipin enthaltende Zubereitung innerhalb der ersten 15 Minuten nach sublingualer Applikation, wobei Nifedipinserumwerte gemessen wurden. In Fig. 4 sind die aus den Werten der drei Probanden gemäß Fig. 1, 2 und 3 ermittelten Mittelwerte gesondert dargestellt. Die Fig. 5 bis 7 zeigen die relative Bioverfügbarkeit für eine zweite Nifedipin enthaltende Zubereitung innerhalb von acht Stunden, wobei Nifedipinserumwerte gemessen wurden. In Fig. 8 sind die aus den Werten der drei Probanden gemäß Fig. 5, 6 und 7 ermittelten Mittelwerte gesondert dargestellt. Die jeweils gemessenen Werte sind hiebei in Nanogramm je Milliliter auf der Ordinate gegen die auf der Abszisse aufgetragene Zeit dargestellt. Die Fig. 9 bis 13 zeigen die Extinktionen von Nifedipin enthaltenden Sprays, denen Polyvinylpyrrolidon bzw. Copolyvidon in unterschiedlichen Mengen zugesetzt wurde, nach Applikation in Wasser.

Als Vergleichsprobe wurde in den Fig. 1 bis 4 eine Zubereitung herangezogen, welche insgesamt in 150 mg, 5 mg Nifedipin, 40 mg Glycerin-Polyäthylenglykoloxystearat, 50 mg Äthanol, 0,1 mg Pfefferminzöl, 0,3 mg Glycerin, 0,05 mg Natriumsaccharinat, 0,25 mg Wasser und 54,3 mg eines Treibmittels auf der Basis von halogenierten Fluorkohlenwasserstoffen, enthielt. Dieses Präparat wurde mit Zerbeißkapseln mit Nifedipin verglichen, wie sie handelsüblich und auf dem Markt erhältlich sind. Die Werte, welche sich bei Anwendung von einem sublingual verabreichten Sprühstoß der erfindungsgemäßen Zusammensetzung erzielen ließen, sind jeweils durch die Kurve 1 veranschaulicht, wohingegen die Vergleichswerte nach sublingualer Applikation der Zerbeißkapseln durch die Kurve 2 charakterisiert sind. Es ergibt sich aus diesen Vergleichsversuchen, daß bereits nach 2 Minuten alle Probanden Nifedipine im Serum aufwiesen, wohingegen kein einziger Proband innerhalb dieser kurzen Zeit bei Anwendung von Zerbeißkapseln Serumwerte für Nifedipin erkennen ließ. Nach einem kurzen Rückgang der Konzentration, der auch bei den Zerbeißkapseln verzögert auftritt, zeigt sich ein steter Anstieg, der gegenüber den Zerbeißkapseln deutlich früher erfolgt. Die Erzielung der minimal wirksamen Wirkstoffkonzentrationen wird somit durch die Anwendung von Sprays mit der erfindungsgemäßen Zusammensetzung wesentlich früher erreicht als mit konventionellen Formulierungen.

Als Vergleichsprobe wurde in den Fig. 5 bis 8 eine Sprayzubereitung herangezogen, welche in insgesamt 150 mg, 5 mg Nifedipin, 50 mg Glycerin-Polyäthylenglykoloxystearat, 30 mg Äthanol, 5 mg Copolyvidon und 60 mg eines Treibmittels, auf der Basis von halogenierten Fluorkohlenwasserstoffen, enthielt. Auch bei diesem Versuch wurde die Sprayprobe mit Nifedipinkapseln verglichen, wie sie handelsüblich und auf dem Markt erhältlich sind. Der Spray wurde sublingual verabreicht, während die Kapseln mit Wasser unzerbissen geschluckt wurden. Die Werte, welche sich bei Anwendung von einem Sprühstoß der erfindungsgemäßen Zusammensetzung erzielen ließen, sind jeweils durch die Kurve 1 veranschaulicht, wohingegen die Vergleichswerte durch die Kurve 2 charakterisiert sind. Aus diesen Vergleichsversuchen ergibt sich, daß der erste Proband bereits nach 8 Minuten Nifedipin im Serum aufwies und nach 15 Minuten alle Probanden Werte von über 12 ng/ml Nifedipin im Serum festgestellt wurden. Zu diesem Zeitpunkt konnte erst bei einem Probanden nach Anwendung von Kapseln ein Serumwert für Nifedipin von 1,7 ng/ml gefunden werden. Nach 30 bis 45 Minuten wird sowohl bei Anwendung des Sprays als auch bei den Zerbeißkapseln der Maximalwert der Serumkonzentration erreicht. Nach dem Erreichen der maximalen Wirkstoffkonzentration liegt der Serumwert der in Form von Sprays verabreichten Zusammensetzung, bis zum vollständigen Rückgang der Wirkstoffkonzentration, nach über 8 Stunden, über demjenigen Wert, der durch die Verabreichung von Kapseln erreicht werden kann. Durch die Verabreichung von Sprays der erfindungsgemäßen Zusammensetzung lassen sich bedeutend früher wirksame Nifedipinkonzentrationen erreichen, die auch länger aufrechterhalten werden können. Die Fläche unter der Kurve, die ein Maß für die Menge an wirksamen Arzneistoff im Körper darstellt, ist beim Spray um ca. 40 % größer als bei der Kapsel.

Es ist auch gelungen, abweichend von Beschreibungen in der Literatur die mittleren, maximalen Plasmaspiegel nach der sublingualen Anwendung jenen der oral zu verabreichenden Proben anzugleichen. Dies bedeutet eine schneller einsetzende Wirkung, einen gleich starken therapeutischen Effekt und eine länger anhaltende therapeutische Wirkung.

Weitere Vorteile der erfindungsgemäßen Zusammensetzungen ergeben sich an Hand, von die Gegebenheiten bei sublingualer Verabreichung simulierenden, Vergleichsbeispielen.

Die in den Fig. 9 bis 13 angeführten Beispiele zeigen deutlich die Steuerbarkeit der Wasserlöslichkeit von Nifedipin in flüssigen Darreichungsformen wie z.B. Spray. Durch den Zusatz von PVP bzw. PVP-Copolymeren zu Nifedipin-Lösungen aus Spray kann zwar das Ausfallen von Nifedipinkristallen im Mund nach der Einnahme aller Voraussicht nach, bei Anwesenheit nur geringer Mengen an Lösungsmittel nicht vollständig verhindert werden, jedoch lösen sich diese Kristalle innerhalb kürzester Zeit beim Verdünnen im Wasser bzw. Magensaft wieder auf und stehen damit zur Resorption zur Verfügung. Auf diese Weise wird es möglich, die Unterschiede, die bei dem Wirkstoff im Blutspiegel bei sublingualer und oraler Einnahme auftreten, in die jeweilige gewünschte Charakteristik anzugleichen.

Die Wasserlöslichkeit wurde durch Messung der Extinktion bei 350 nm über die Zeit bestimmt. Zur Simulation der Verhältnisse bei sublingualer Anwendung wurde jeweils in einem der Speichelmenge etwa entsprechenden Volumen gelöst und dieses Volumen 10 min unverändert belassen, worauf anschließend auf 900 ml mit $H_2O$ ergänzt wurde, wobei die Messung nach der in USP Dissulution Test beschriebenen Methode erfolgte. Die Temperatur wurde auf 37°C + 0,5°C konstant gehalten und in den ersten 10 min konnten sich die zu erwartenden Kristalle bei den Zubereitungen ausbilden.

Bei der vergleichenden Analyse von Sprays mit Polyäthylenglykol wurde festgestellt, daß zwei Spraystöße eines 5 mg dosierenden Nifedipinsprays nach 5 min eine Extinktion bei 350 nm von 0,014 und nach 20 min von 0,031 aufweisen. Der Kurvenverlauf war flach und konnte durch Zusatz von 10 mg Copolyvidon deutlich steiler geführt werden. Bei Zusatz von 10 mg Copolyvidon wurden Extinktionswerte nach 5 min von 0,021 und nach 20 min von 0,057 gemessen. Bei den Spraystößen wurde jeweils eine Verdünnung mit Wasser von 1:1 zur Simulation der Verhältnisse bei sublingualer Darreichung vorgenommen.

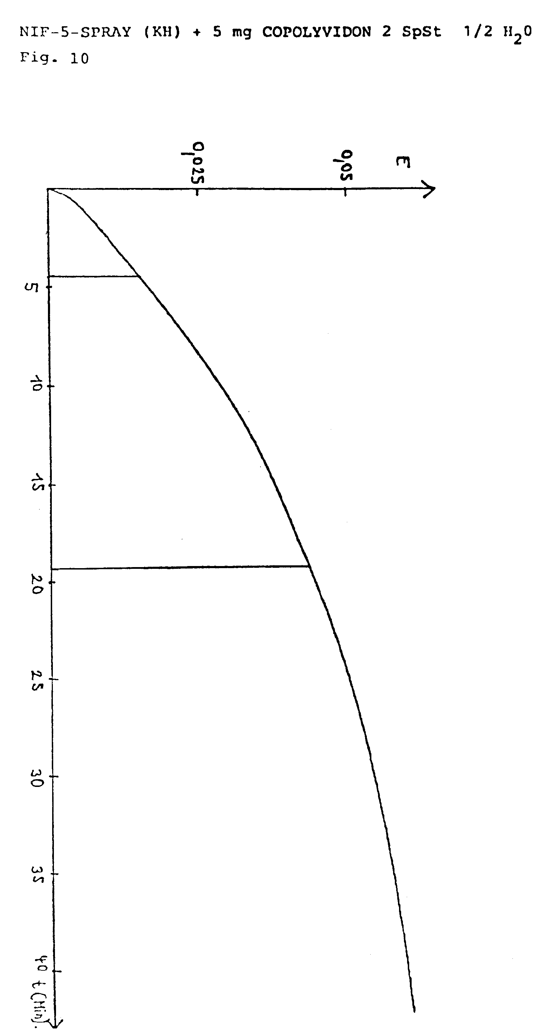

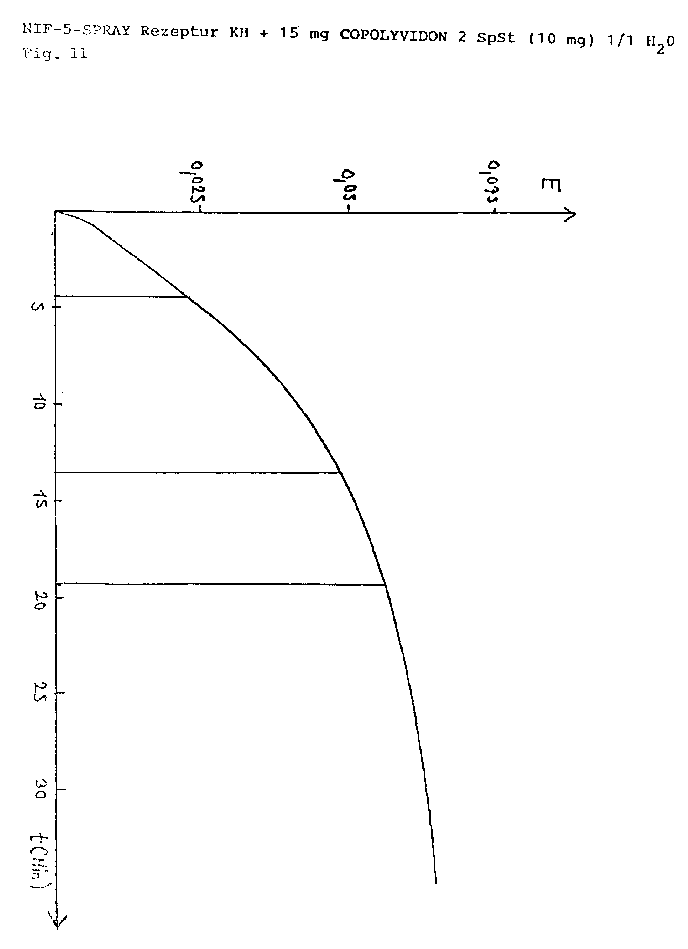

Bei Vergleichsversuchen mit kontinuierlich ansteigendem Zusatz an Copolyvidon wurde herausgefunden, daß ein ohne Zusatz von Copolyvidon verabreichter Spray gleicher Wirkstoffmenge eine Extinktion von 0,013 nach 5 min und 0,026 nach 20 min zeigte. Nach Zusatz von 5 mg Copolyvidon wurden Werte von 0,020 nach 5 min und 0,045 nach 20 min und damit bereits ein deutlich steilerer Verlauf der Kurve erzielt. Bei Zusatz von 10 mg Copolyvidon wurden nach 5 min Extinktionswerte von 0,020 und nach 20 min von 0,052 gemessen, wohingegen bei Zusatz von 15 mg Copolyvidon die Werte bereits deutlich ansteigen und nach 5 min eine Extinktion von 0,024 und nach 20 min eine Extinktion von 0,058 gemessen werden konnte. Schließlich wurde bei Zusatz von 20 mg Copolyvidon ein Extinktionswert nach 5 min mit 0,033 und nach 20 min mit 0,078 bestimmt. Die Verhältnisse für einen Spray ohne Zusatz von Copolyvidon sind hiebei in Fig. 9 dargestellt. Die deutliche länderung der Kurve nach Zusatz von 5 mg Copolyvidon zu einem Spray gemäß Fig. 9 ist in Fig. 10 ersichtlich. Schließlich zeigt Fig. 11 den typisch steileren Kurvenverlauf bei Zusatz von 15 mg Copolyvidon.

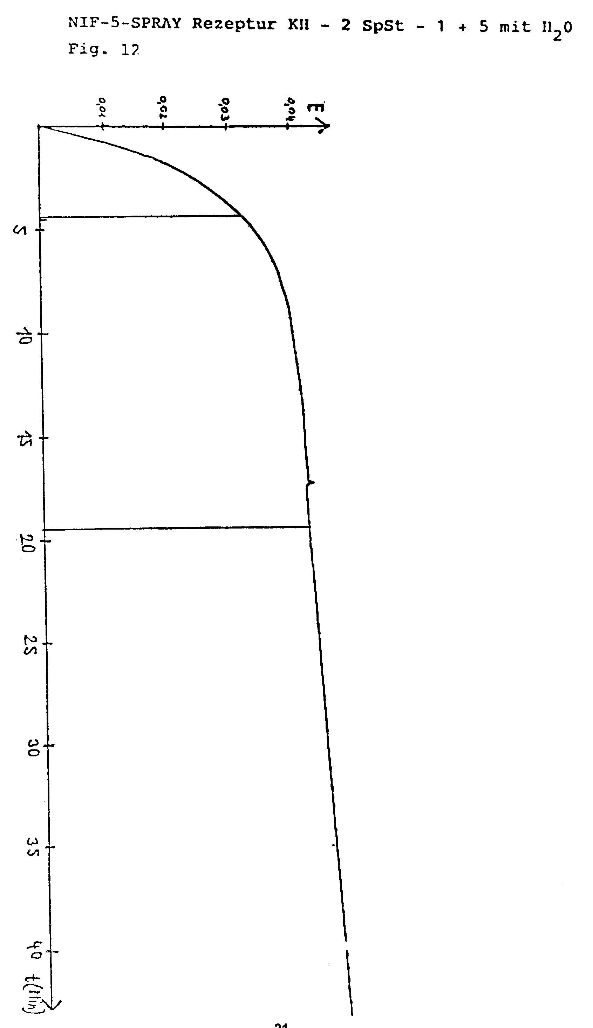

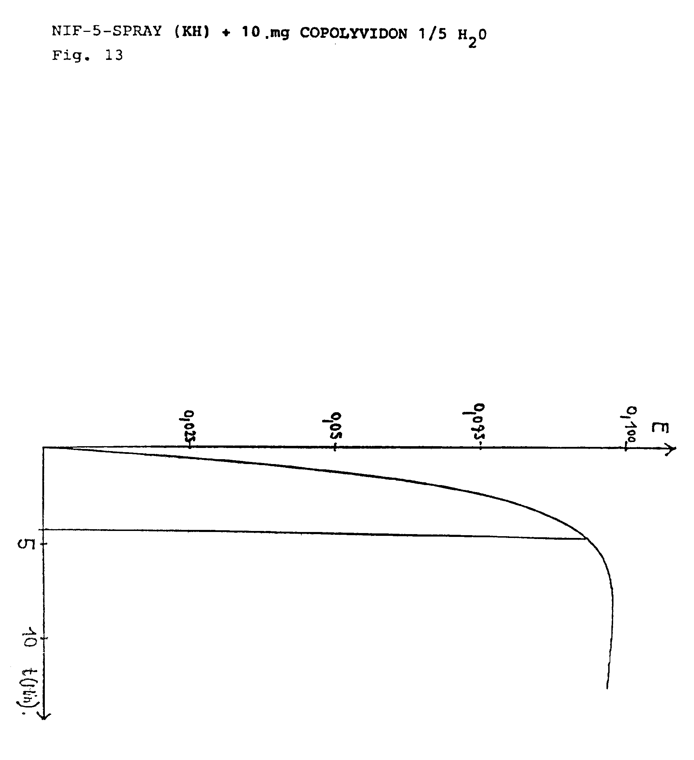

Bei Verdünnung eines derartigen Sprays mit Wasser im Volumsverhältnis 1:5 bezogen auf das Volumen des Maxspraystoßes wurde ohne Zusatz von Copolyvidon eine Extinktion von 0,034 nach 5 min und nach 20 min von 0,045 und damit ein flacher Kurvenverlauf gemessen. Dies ist in Fig. 12 dargestellt. Nach Zusatz von 10 mg Copolyvidon ergab sich der Kurvenverlauf gemäß Fig. 13, wobeinach 5 min bereits eine Extinktion von 0,095 und nach 6 min das Maximum bei 0,098 festgestellt werden konnte.

Auch eine entsprechend verbesserte Rezeptur für Sprays mit einem Glycerin-Polyäthylenglykoloxysstearat zeigte eine deutliche Verbesserung und eine Konzentrationsabhängigkeit der Verbesserung in Abhängigkeit von der Menge zugesetztem Copolyvidon. Ohne Zusatz von Copolyvidon konnten Extinktionswerte von 0,028 nach 5 min und 0,049 nach 20 min und damit ein mittlerer Kurvenverlauf erzielt werden. Durch Zusatz von 10 mg Copolyvidon erhöhten sich die Werte auf 0,036 nach 5 min und auf 0,056 nach 20 min. Bei Zusatz von 5 mg Copolyvidon konnte bereits nach 2 min ein Maximum von 0,072 gemessen werden, womit ein deutlich steilerer Kurvenverlauf die Folge ist. Analoge Ergebnisse werden mit Zusammensetzungen erzielt, bei welchen das Copolyvidon durch entsprechende Mengen an Polyvinylpyrrolidon oder Copolymere aus Acrylsäureäthylester und Methacrylsäure oder Gemischen davon ersetzt wird.

Beispiel 1:

Vier Nifedipin enthaltende Wirkstoffzusammensetzungen wurden in einem Versuch, bei welchem die Verhältnisse bei sublingualer Anwendung simuliert wurden, dahingehend untersucht, wie lange die Zusammensetzungen unter diesen Versuchsbedingungen stabil sind.

|  | 1.Zus. | 2.Zus. | 3.Zus. | 4.Zus. |
|---|---|---|---|---|
| Nifedipin | 5 mg | 5 mg | 5 mg | 5 mg |
| Glycerin-Poly-äthylenglykol-oxystearat | 50 mg | 50 mg | 50 mg | 45 mg |
| Äthanol | 30 mg | 30 mg | 25 mg | 30 mg |
| Treibgas | 65 mg | 60 mg | 60 mg | 60 mg |
| Copolyvidon | -- | 5 mg | 10 mg | 10 mg |
|  | 150 mg | 150 mg | 150 mg | 150 mg |

Es werden in einem Uhrglas jeweils 0,1 ml Wasser vorgelegt, ein Sprühstoß von 160 mg abgegeben und nach 30 Sekunden weitere 0,2 ml Wasser zugesetzt.

Bei der 1. Zusammensetzung wird 4 Minuten nachdem Wasser zugesetzt wurde, eine Trübung der Lösung durch ausfallendes Nifedipin festgestellt.

Bei der 2. Zusammensetzung werden nach 30 Minuten am Glasboden kleine Nifedipinkristalle abgeschieden.

Aus der 3. und 4. Zusammensetzung fällt erst nach 50 Minuten Nifedipin aus; wobei bei diesen Zusammensetzungen die Zusammensetzung 3 etwas stabiler als die Zusammensetzung 4 ist, da die sich abscheidende Nifedipinmenge im Falle der Zusammensetzung 3 bedeutend geringer ist.

Aus diesen Ergebnissen zeigt sich, daß die Zusammensetzungen 2 bis 4 für sublinguale Anwendung geeignet sind, wobei die Zusammensetzung 3, auf Grund ihrer überaus großen Stabilität am besten geeignet ist. Dies wird auf das Vorhandsein von gleichzeitig relativ großen Mengen von Copolyvidon und Glycerin-Polyäthylenglykoloxystearat zurückgeführt. Analoge Ergebnisse werden mit Zusammensetzungen erzielt, bei welchen das Copolyvidon durch entsprechende Mengen an Polyvinylpyrrolidon oder Copolymere aus Acrylsäureäthylester und Methacrylsäure oder Gemischen davon ersetzt wird.

Beispiel 2:

In vergleichenden Versuchen werden vier Nifedipin-Wirkstoffzusammensetzungen, die jeweils Propylencarbonat als Fällungshemmer bei Einbringung in den Speichel enthalten, auf ihr Lösungsverhalten in Wasser und damit auf ihre sublinguale Anwendbarkeit untersucht.

|  | 1.Zus. | 2.Zus. | 3.Zus. | 4.Zus. |
|---|---|---|---|---|
| Nifedipin | 5 mg | 5 mg | 5 mg | 5 mg |
| Äthanol | 20 mg | 10 mg | 10 mg | -- |
| Propylencarbonat | 40 mg | 50 mg | 30 mg | 50 mg |
| Polyäthylenglykol | 20 mg | -- | -- | -- |
| mittelkettige Fettsäure-triglyceride | -- | 40 mg | 35 mg | 50 mg |
| Glycerin-Polyäthylen-glykoloxystearat | -- | -- | 10 mg | -- |
| Treibgas | 65 mg | 50 mg | 60 mg | 50 mg |
|  | 150 mg | 155 mg | 150 mg | 155 mg |

Von diesen Zusammensetzungen wird jeweils ein Sprühstoß von 150 mg, auf 0,3 ml in einem Uhrglas vorgelegtes Wasser abgegeben und in einem zweiten Versuch erst auf das trockene Uhrglas ein Sprühstoß der Wirkstoffzusammensetzung ausgeführt und dann 0,5 ml Wasser zugegeben.

In beiden Fällen wurde beobachtet, ob Nifedipin aus der öligen, klaren Zusammensetzung ausfällt oder nicht.

Aus der 1. Zusammensetzung fällt Nifedipin in beiden Fällen sofort aus, die 2. Zusammensetzung bleibt hingegen bei beiden Versuchen über einen Zeitraum von 20 Minuten eine klare, ölige Flüssigkeit.

Aus der 3. Zusammensetzung fällt beim ersten Versuch (Wasser wird vorgelegt) sofort Nifedipin aus, beim zweiten Versuch bleibt die Lösung klar.

Die 4. Zusammensetzung bleibt ebenso wie die 2. Zusammensetzung bei beiden Versuchen über einen Zeitraum von mindestens 20 min eine klare, ölige Flüssigkeit.

Aus diesen Ergebnissen kann man erkennen, daß die 2. und 4. Zusammensetzung, das sind diejenigen mit dem höchsten Anteil an Propylencarbonat für eine sublinguale Anwendung am besten geeignet sind.

Beispiel 3:

Drei Nifedipin enthaltende Wirkstoffzusammensetzungen wurden in einem Versuch, bei welchem die Verhältnisse bei sublingualer Anwendung simuliert wurden, dahingehend untersucht, wie lange die Zusammensetzungen unter diesen Versuchsbedingungen stabil sind.

|  | 1.Zus. | 2.Zus. | 3.Zus. |
|---|---|---|---|
| Nifedipin | 5 mg | 5 mg | 5 mg |
| Copolymer aus Methacrylsäure und Acrylsäureätyhlester | - | 5 mg | 5 mg |
| Glycerin-Polyäthylenglykoloxystearat | 50 mg | 50 mg | 40 mg |
| Äthanol | 30 mg | 40 mg | 50 mg |
| Treibgas | 65 mg | 60 mg | 60 mg |
|  | 150 mg | 160 mg | 160 mg |

Von diesen Zusammensetzungen wird jeweils ein Sprühstoß von 150 mg, auf 0,3 ml in einem Uhrglas vor-

EP 0 240 484 B1

gelegtes Wasser abgegeben und in einem zweiten Versuch erst auf das trockene Uhrglas ein Sprühstoß der Wirkstoffzusammensetzung ausgeführt und dann 0,5 ml Wasser zugegeben.

In beiden Fällen wurde beobachtet, ob Nifedipin aus der öligen, klaren Zusammensetzung ausfällt oder nicht.

Bei der ersten Zusammensetzung wird bei beiden Versuchen etwa 4 Minuten nach dem Versprühen eine Trübung beobachtet.

Aus der zweiten und dritten Zusammensetzung fällt beim ersten Versuch (Wasser wird vorgelegt) sofort ein feinkristalliner Niederschlag von Nifedipin aus, der sich jedoch bei weiterer Verdünnung mit Wasser wieder auflöst.

Beim zweiten Versuch wird nach dem Versprühen kein Ausfallen von Nifedipin beobachtet, erst nach Wasserzugabe fällt Nifedipin aus, worauf sich die Kristalle bei weiterer Verdünnung wiederum auflösen.

Aus diesen Ergebnissen kann man erkennen, daß die zweite und dritte Zusammensetzung für sublinguale Applikation geeignet sind, da durch den Speichel eine permanente Verdünnung mit Wasser und dadurch ein rasches Wiederauflösen der ausgefallenen Nifedipinkristalle eintritt.

Ein weiteres Ausführungsbeispiel für eine erfindungsgemäße Rezeptur besteht darin, daß 5 mg Nifedipin, 70 mg Glycerin-Polyäthylenglykoloxystearat und 30 mg Äthanol gemeinsam mit 55 mg eines Treibmittels auf der Basis halogenierter Kohlenwasserstoffe zur Erzielung eines dosierten Sprühstrahles vermengt wurden.

Zur Erzielung eines 10 mg-Sprays wurde eine entsprechend höher dosierendes Ventil eingesetzt und es wurde mit Vorteil eine Zusammensetzung gewählt, welche 5,5 mg Nifedipin, 40 mg Glycerin--Polyäthylenglykoloxystearat, 49,5 mg Äthanol, Pfefferminzöl, Glycerin, Natriumsaccharat in üblichen Mengen sowie 0,25 mg Wasser und 54,3 mg eines Treibmittels auf Basis von halogenierten Kohlenwasserstoffen je 150 mg enthielt. Diese Zusammensetzung wurde mit einem Sprühstoß von 270 mg ausgebracht, wodurch sich eine Dosierung von 10 mg Nifedipin je Sprühstoß ergab.

In allen Fällen konnten auch bei tiefen Temperaturen überaus stabile Zusammensetzungen erzielt werden, welche neben einer besonders verbesserten Stabilität eine besonders rasche Ansprechcharakteristik zeigten.


**Patentansprüche**

1. Pharmazeutische Zubereitung mit Nifedipin als Wirkstoff, wobei der Wirkstoff zusammen mit 1) Polyalkoholen, wie Polyäthylen- und/oder Polyalkylenglykolen und/oder Alkylphenyläthern des Polyäthylenglykols und/oder Polyglycerinfettsäureestern und/oder Glycerin-Polyäthylenglykoloxyfettsäureestern wie Glycerin-Polyäthylenglykoloxyoleat und/oder Glycerin-Polyäthylenglykoloxystearat und/oder partiellen Fettsäureestern des Sorbitans bzw. des Polyhydroxyäthylensorbitans und/oder Polyvinylalkohols und/oder Polyhydroxyäthylenfettalkoholäthern bzw. Polyhydroxyäthylenfettsäureestern und/oder Polyhydroxyäthylenpolyhydroxypropylenkondensaten und/oder mit 2) Copolymeren aus Acrylsäure und Methacrylsäure, und/oder Copolymeren aus Methacrylsäureestern und Acrylsäureestern, und/oder Copolymeren aus Methacrylsäure und Methacrylsäuremethylester und 3) Äthanol, gelöst vorliegt, wobei das Gewichtsverhältnis von Nifedipin und den Verbindungen 1) und 2) 1:2 bis 1:25 beträgt, wobei 4) als Fällungshemmer bei Einbringung in den Speichel Copolyvidon oder Polyvinylpyrrolidon oder Copolymere aus Methacrylsäure und Acrylsäureäthylester in Mengen von 25 bis 300 Gew.-%, insbesondere 50 bis 200 Gew.-%, bezogen auf die Menge an Nifedipin oder Propylencarbonat verwendet wird, die Zusammensetzung zusätzlich Äthanol, gegebenenfalls wenigstens teilweise ersetzt durch mittelkettige Fettsäuredi- und/oder -triglyceride im Verhältnis von 1:25 bis 1:4 Gewichtsteilen Nifedipin : Äthanol und ein Treibgas enthält und wobei weiters in der Zusammensetzung maximal 7,5 Gew.-% Wasser in bezug auf das Gesamtgewicht der Lösung enthalten sein können.

2. Flüssige pharmazeutische Zubereitung nach Anspruch 1 für Verabreichung als Aerosol mit Nifedipin als Wirkstoff, einem geeigneten Lösungsmittel und einem in einem Spraybehälter enthaltenen Treibmittel, wobei das Lösungsmittel Äthanol und einen Polyalkohol enthält, wobei gegebenenfalls zumindest ein Teil des Äthanols durch mittelkettige Fettsäuretriglyceride ersetzt ist, und ein oder mehrere als Fällungshemmer bei Einbringung in den Speichel wirkende Substanzen enthalten sind, die aus der Gruppe der Polyalkohole, Polyvinylpyrrolidon, Alkylencarbonate, Copolyvidon und Copolymere aus Acrylsäureäthylester und Methacrylsäure gewählt sind, wobei das Mengenverhältnis Nifedipin : Äthanol bzw. mittelkettigen Fettsäuretriglyceriden im Bereich von 1:25 bis 1:4 liegt und das Mengenverhältnis Nifedipin : Polyalkohol im Bereich von 1:2 bis 1:25 liegt, mit dem Proviso, daß

a) wenn der Polyalkohol Polyäthylenglykol ist, zusätzlich ein oder mehrere andere Polyalkohole enthalten sind und/oder ein wie oben definierter Fällungshemmer bei Einbringung in den Speichel und/oder das Äthanol zumindest teilweise durch mittelkettige Fettsäuretriglyceride ersetzt ist und

b) wenn der Polyalkohol Glycerin-Polyäthylenglykoloxystearat ist, das Gewichtsverhältnis Treibmittel :

Nifedipin unter 220 liegt und/oder das Äthanol zumindest teilweise durch mittelkettige Fettsäuretriglyceride ersetzt ist und wobei weiters

c) in der Zusammensetzung maximal 7,5 Gew.-% Wasser in bezug auf das Gesamtgewicht der Lösung enthalten sein können.

3. Zubereitung nach Anspruch 1, dadurch gekennzeichnet, daß bei Verwendung von Propylencarbonat als Fällungshemmer bei Einbringung in den Speichel zur Herstellung einer stabilen, sprühfähigen Zusammensetzung, 25 bis 40 %, insbesondere 30 bis 35 Gew.-%, Treibgas enthalten sind.

4. Zubereitung nach einem der Ansprüche 1, 2 oder 3, dadurch gekennzeichnet, daß bei Verwendung von Propylencarbonat als Fällungshemmer bei Einbringung in den Speichel, Äthanol zumindest teilweise durch mittelkettige Fettsäuretriglyceride ersetzt wird.

5. Zubereitung nach Anspruch 3, dadurch gekennzeichnet, daß das Verhältnis Nifedipin : Propylencarbonat im Bereich 1:6 bis 1:10 und das Verhältnis Nifedipin : mittelkettigen Fettsäuretriglyceriden im Bereich zwischen 1:6 und 1:10 liegt.

6. Zubereitung nach einem der Ansprüche 1 oder 2, dadurch gekennzeichnet, daß ein Copolymer aus 60 % Polyvinylpyrrolidon und 40 % Polyvinylacetat eingesetzt wird.

7. Zubereitung nach Anspruch 1 oder 2, dadurch gekennzeichnet, daß die Zusammensetzung in 150 mg, 5 mg Nifedipin, 40 bis 55 mg Glycerin-Polyäthylenglykoloxystearat, 20 bis 35 mg Äthanol, 5 bis 15 mg Copolyvidon oder Polyvinylpyrrolidon oder Polyacrylsäure oder Gemische davon und 55 bis 70 mg Treibgas enthält.

8. Verfahren zur Herstellung der Zubereitungen mit Nifedipin als Wirkstoff nach Anspruch 1, wobei Nifedipin zusammen mit 1) Polyalkoholen, wie Polyäthylen- und/oder Polyalkylenglykolen und/oder Alkylphenyläthern des Polyäthylenglykols und/oder Polyglycerinfettsäureestern und/oder Glycerin-Polyäthylenglykoloxyfettsäureestern wie Glycerin-Polyäthylenglykoloxyoleat und/oder Glycerin-Polyäthylenglykoloxystearat und/oder partiellen Fettsäureestern des Sorbitans bzw. des Polyhydroxyäthylensorbitans und/oder Polyvinylalkohols und-/oder Polyhydroxyäthylenfettalkoholäthern bzw. Polyhydroxyäthylenfettsäureestern und/oder Polyhydroxyäthylenpolyhydroxypropylenkondensaten und/oder mit 2) Copolymeren aus Acrylsäure und Methacrylsäure, und/oder Copolymeren aus Methacrylsäureestern und Acrylsäureestern, und/oder Copolymeren aus Methacrylsäure und Methacrylsäuremethylester und 3), Äthanol gelöst wird, wobei das Gewichtsverhältnis von Nifedipin und den Verbindungen 1) und 2) 1:2 bis 1:25 beträgt, wobei 4) als Fällungshemmer bei Einbringung in den Speichel Copolyvidon oder Polyvinylpyrrolidon oder Copolymere aus Methacrylsäure und Acrylsäureäthylester in Mengen von 25 bis 300 Gew.-%, insbesondere 50 bis 200 Gew.-%, bezogen auf die Menge an Nifedipin oder Propylencarbonat verwendet wird, und zusätzlich Äthanol, gegebenenfalls wenigstens teilweise ersetzt durch mittelkettige Fettsäuredi- und/oder -triglyceride im Verhältnis von 1:25 bis 1:4 Gewichtsteilen Nifedipin : Äthanol und ein Treibgas zugesetzt wird und wobei weiters in der Zusammensetzung maximal 7,5 Gew.-% Wasser in bezug auf das Gesamtgewicht der Lösung zugesetzt werden und gegebenenfalls mit Hilfsstoffen in lichtgeschützte Spraydosen gefüllt wird.

## Claims

1. Pharmaceutical composition, comprising nifedipine as the active ingredient, wherein the active ingredient is dissolved together with 1) polyalcohols, such as polyethylene- and/or polyalkylene glycols and/or alkylphenyl ethers of polyethylene glycol and/or polyglycerine fatty acid esters and/or glycerine-polyethylene glycoloxy fatty acid esters, such as glycerine-polyethylene glycoloxy oleate and/or glycerine-polyethylene glycoloxy stearate and/or partial fatty acid esters of sorbitol or polyhydroxy ethylene sorbitol and/or polyvinyl alcohols and/or polyhydroxy ethylene fatty alcohol ethers or polyhydroxy ethylene fatty acid esters and/or polyhydroxy ethylene-polyhydroxy propylene condensates and/or 2) acrylic acid-methacrylic acid copolymers and/or methacrylic acid esters-acrylic acid esters copolymers and/or methacrylic acid-methacrylic acid methyl ester copolymers and 3) ethanol, wherein the weight ratio of nifedipine and of the compounds 1) and 2) is between 1:2 and 1:25, wherein 4) copolyvidone or polyvinyl pyrrolidone or methacrylic acid-acrylic acid ethyl ester copolymers are used as precipitation blocking agent when introducing in the saliva in amounts of 25 to 300 weight%, particularly 50 to 200 weight%, relative to the content of nifedipine or polypropylene carbonate, the composition containing additionally ethanol, which is optionally at least partially replaced by a medium-chain fatty aciddi- and/or -triglyceride in a weight ratio of 1:25 to 1:4 nifedipine:ethanol and a propelling agent and wherein additionally the maximum water content in the composition may be 7,5 weight% relative to the total weight of the solution.

2. Liquid pharmaceutical composition according to claim 1 suitable for the aerosol administration, comprising nifedipine as the active ingredient, a suitable solvent and a propelling agent held within an aerosol-dispensing container, wherein the solvent contains ethanol and a polyalcohol, wherein optionally at least part of the

EP 0 240 484 B1

ethanol is replaced by medium-chain fatty acid triglyceride, and one or more substances are contained acting as precipitation blocking agents when introducing in the saliva, which are selected from the group consisting of polyalcohols, polyvinyl pyrrolidone, alkylene carbonate, copolyvidone and acrylic acid ethylester-methacrylic acid copolymers, wherein the ratio of nifedipine:ethanol or medium-chain fatty acid triglyceride is in the range of 1:25 to 1:4 and the ratio of nifedipine: polyalkohols is in the range of 1:2 to 1: 25, provided that

a) when the polyalkohol is polyethylene glycol, there are additionally present one or more other polyalkohols and/or a precipitation blocking agent as defined above when introducing in the saliva and/or there is at least part of the ethanol replaced by a medium-chain fatty acid triglyceride and

b) when the polyalcohol is glycerine-polyethylene glycoloxy stearate, the ratio of propelling agent:nifedipine is less than 220 and/or at least part of the ethanol is replaced by medium-chain fatty acid triglycerides, and wherein additionally

c) the maximum water content in the composition may be 7,5 weight% relative to the total weight of the solution.

3. Composition according to claim 1, characterized in that 25 to 40 weight%, in particular 30 to 35 weight%, propelling agent are present for the preparation of a stable, sprayable composition, when propylene carbonate is used as the precipitation blocking agent when introducing in the saliva.

4. Composition according to any of the claims 1, 2 or 3, characterized in that at least part of the ethanol is replaced by medium-chain fatty acid triglycerides, when propylene carbonate is used as the precipitation blocking agent when introducing in the saliva.

5. Composition according to claim 3, characterized in that the ratio of nifedipine:propylene carbonate is in the range of 1:6 to 1:10 and the ratio of nifedipine:medium-chain fatty acid triglycerides is in the range of 1:6 to 1:10.

6. Composition according to any of the claims 1 or 2, characterized in that a copolymer of 60% polvinyl pyrrolidone and 40% polyvinyl acetate is used.

7. Composition according to any of the claims 1 or 2, characterized in that 150 mg of the composition contains 5 mg nifedipine, 40 to 55mg glycerine-polyethylene glycoloxy stearate, 20 to 35mg ethanol, 5 to 15mg copolyvidone or polyvinyl pyrrolidone or polyacrylic acid or mixtures thereof, and 55 to 70mg propelling agent.

8. Process for the manufacture of compositions containing nifedipine according to claim 1, comprising the dissolution of nifedipine together with 1) polyalcohols, such as polyethylene- and/or polyalkylene glycols and/or alkylphenyl ethers of polyethylene glycol and/or polyglycerine fatty acid esters and/or glycerine-polyethylene glycoloxy fatty acid esters such as glycerine-polyethylene glycoloxy oleate and/or glycerine- polyethylene glycoloxy stearate and/or partial fatty acid esters of sorbitol or polyhydroxy ethylene sorbitol and/or polyvinyl alcohols and/or polyhydroxy ethylene fatty alcohol ethers or polyhydroxy ethylene fatty acid esters and/or polyhydroxy ethylene-polyhydroxy propylene condensates and/or 2) acrylic acid-methacrylic acid copolymers and/or methacrylic acid ester-acrylic acid ester copolymers and/or methacrylic acid-methacrylic acid methyl ester copolymers and 3) ethanol, wherein the weight ratio of nifedipine and of the compounds 1) and 2) is between 1:2 and 1:25, wherein 4) copolyvidone or polyvinyl pyrrolidone or methacrylic acid-acrylic acid ethyl ester copolymers are used as precipitation blocking agent when introducing in the saliva in amounts of 25 to 300 weight%, particularly 50 to 200 weight%, relative to the content of nifedipine or polypropylene carbonate, and wherein additionally ethanol which is optionally at least partially replaced by a medium-chain fatty aciddi- and/or -triglyceride in a weight ratio of 1:25 to 1:4 nifedipine:ethanol and a propelling agent is added, and wherein additionally a maximum water content of 7,5 weight% relative to the composition is added, and the composition optionally together with adjuvants is introduced in a light-protected aerosol can.

**Revendications**

1. Composition pharmaceutique avec nifédipine comme matière active, où la matière active est dissoute conjointement avec 1) polyalcools, comme polyéthylène- et/ou polyalcylène-glycols et/ou éthers alkylphényliques du polyéthylèneglycol et/ou esters polyglycériniques de l'acide gras et/ou esters polyéthylèneglycoloxy-glycériniques de l'acide gras comme l'ester polyéthylèneglycoloxy-glycérinique de l'acide oléique et/ou l'ester polyéthylèneglycoloxy-glycérinique de l'acide stéarique et/ou esters de l'acide gras partielle de sorbitane ou de polyhydroxyéthylènesorbitane et/ou l'alcool polyvinylique et/ou ethers polyhydroxyéthyléniques de l'alcools gras ou esters polyhydroxyéthyléniques de l'acide gras et/ou condensates polyhydroxyéthyléniques-polyhydroxypropyléniques et/ou 2) copolymèrs de l'acide acrylique et de l'acide méthacrylique et/ou copolymèrs de l'esters de l'acide méthacrylique et de l'esters de l'acide acrylique et/ou copolymèrs de l'acide méthacrylique et de l'ester méthylique de l'acide méthacrylique et 3) éthanol, où la proportion du poids du nifédipine et les components 1) et 2) est entre 1:2 et 1:25 où 4) copolyvidone ou polyvinylpyrrolidone ou copolymèrs de l'acide

11

méthacrylique et de l'ester éthylique de l'acide acrylique sont usés comme arrêt de précipitation en introduissant dans la salive, dans une quantité de 25 à 300% en poids, en particulière de 50 à 200 % en poids, respective à la masse de nifédipine ou propylènecarbonate, la composition contiens en outre éthanol, qui est, le cas échéant, remplacé au moins à part par des diglycérides de l'acide gras à medium chaine et/ou des triglycérides de l'acide gras à medium chaine en proportion de 1:25 à 1:4 parts à poids nifédipine :éthanol et un propellant et où en outre le contenu maximal de l'eau dans la composition peut être 7,5 % en poids relative au poids total de la solution.

2. Composition pharmaceutique liquide selon la revendication 1 convenable à l'administration comme aérosol avec nifédipine comme matière active, un solvant convenable et un propulseur contenu dans un récipient aérosol, où le solvant contient éthanol ou un polyalcool, où, le cas échéant, au moins un part du éthanol est remplacé par des triglycérides de l'acide gras à medium chaine et une ou plusieurs substances sont usées comme arrêt de précipitation en introduissant dans la salive, qui sont selectionnées de la groupe consistant de polyalcools, polyvinylpyrrolidone, alkylènecarbonates, copolyvidone et copolymères de l'ester éthylique de l'acide acrylique et de l' acide méthacrylique, où la proportion des masses nifédipine:éthanol ou triglycérides de l'acide gras à medium chaine est environ de 1:25 à 1:4 et la proportion des masses nifédipine:polyalcool est environ de 1:2 à 1:25, sous réserve que

a) quand le polyalcool est polyéthylèneglycol, il y a en addition un ou plusieurs autre polyalcools et/ou un arrêt de précipitation en introduissant dans la salive comme décrite dessus et/ou l'éthanol est remplacé au moins à part par des triglycérides de l'acide gras à medium chaine et

b) quand le polyalcool est l'ester polyéthylèneglycoloxyglycérinique de l'acide stéarique, la proportion du poids propellant:nifédipine est sous 220 et/ou l'éthanol est remplacé au moins à part par des triglycérides de l'acide gras à medium chaine et où en outre

c) le contenu maximal de l'eau dans la composition peut être 7,5 % en poids relative au poids total de la solution.

3. Composition selon la revendication 1, characterisée en ce que de 25 à 40 % en poids, en particulière de 30 à 35 % en poids propellant sont présents pour la préparation d'une composition stable et dispersable, en utilisant du propylènecarbonate comme arrêt de précipitation en introduissant dans la salive.

4. Composition selon une des revendications 1, 2 ou 3, characterisée en ce que l'éthanol est remplacé au moins à part par des triglycérides de l'acide gras à medium chaine, en utilisant du propylènecarbonate comme arrêt de précipitation en introduissant dans la salive.

5. Composition selon la revendication 3, characterisée en ce que la proportion nifédipine:propylènecarbonate est environ de 1:6 à 1:10 et la proportion nifédipine:triglycérides de l'acide gras à medium chaine est environ de 1:6 à 1:10.

6. Compositon selon une des revendications 1 ou 2, characterisée en ce qu'un copolymère de 60% polyvinyl-pyrrolidone et 40% polyvinylacetate est usé.

7. Compositon selon une des revendications 1 ou 2, characterisée en ce que la composition contiens en 150mg, de 5mg nifédipine, de 40 à 55mg l'ester polyéthylèneglycoloxy-glycérinique de l'acide stéarique, de 20 à 35mg éthanol, de 5 à 15mg copolyvidone ou polyvinylpyrrolidone ou l'acide polyacrylique ou des mixtures de ces composants et de 55 à 70mg propellant.

8. Procédé pour la préparation des compositions avec nifédipine comme matière active selon la revendication 1, où nifédipine avec 1) polyalcools, comme polyéthylène et/ou polyalcylèneglycols et/ou éthers alkylphényliques du polyéthylèneglycol et/ou esters polyglycériniques de l'acide gras et/ou esters polyéthylèneglycoloxy-glycériniques de l'acide gras, comme l'ester polyéthylèneglycoloxy-glycérinique de l'acide oléique et/ou l'ester polyéthylèneglycoloxyglycérinique de l'acide stéarique et/ou esters de l'acide gras partielle de sorbitane ou de polyhydroxyéthylènesorbitane et/ou l'alcool polyvinylique et/ou ethers polyhydroxyéthylèniques de l'alcools gras ou esters polyhydroxyéthylèniques de l'acide gras et/ou condensates polyhydroxyéthylèniques-polyhydroxypropylèniques et/ou 2) copolymèrs de l'acide acrylique et de l'acide méthacrylique et/ou copolymèrs de l'esters de l'acide méthacrylique et de l'esters de l'acide acrylique et/ou copolymèrs de l'acide méthacrylique et de l'esters méthylique de l'acide méthacrylique et 3) éthanol, où la proportion du poids du nifédipine et les components 1) et 2) est entre 1:2 et 1:25, où 4) copolyvidone ou polyvinylpyrrolidone ou copolymèrs de l'acide méthacrylique et de l'ester éthylique de l'acide acrylique sont usés comme arrêt de précipitation en introduissant dans la salive, dans une quantité de 25 à 300% en poids, en particulière de 50 à 200 % en poids, respective à la masse de nifédipine ou propylènecarbonate, et la composition contiens en outre éthanol, qui est, le cas échéant, remplacé au moins à part par des diglycérides de l'acide gras à medium chaine et/ou des triglycérides de l'acide gras à medium chaine en proportion de 1:25 à 1:4 parts à poids nifédipine:éthanol et un propellant et en outre un maximum de 7,5 % en poids de l'eau, relative au poids total de la solution, est introduit dans la composition et la composition est introduite, le cas écheant, avec des matières consommables dans des boîtes aérosol protectées contre la lumière.

FIG. 1

FIG. 2

FIG. 3

FIG. 4

Fig. 5

NIFEDIPIN (ng/ml)

**Fig. 7**

ZEIT(min)

Fig. 8
NIFEDIPIN (ng/ml)

NIF-5-SPRAY ohne COPOLYVIDONZUSATZ 2 SpSt ( 10 mg) 1/1 H$_2$0

Fig. 9

18

NIF-5-SPRAY (KH) + 5 mg COPOLYVIDON 2 SpSt 1/2 $H_2O$

Fig. 10

NIF-5-SPRAY Rezeptur KH + 15 mg COPOLYVIDON 2 SpSt (10 mg) 1/1 $H_2O$
Fig. 11

NIF-5-SPRAY Rezeptur KII - 2 SpSt - 1 + 5 mit $H_2O$

Fig. 12

NIF-5-SPRAY (KH) + 10 .mg COPOLYVIDON 1/5 $H_2O$

Fig. 13